# EUROPEAN PATENT SPECIFICATION

(11) **EP 3 654 929 B1**
(45) Date of publication and mention of the grant of the patent: **29.05.2024**
(21) Application number: 18739861.5
(22) Date of filing: 16.07.2018
(51) Int. Cl.: A61K 8/34, A61K 8/49, A61Q 7/00, A61K 8/81

(54) **AQUEOUS COSMETIC COMPOSITION COMPRISING A PYRIDINEDICARBOXYLIC ACID DERIVATIVE AND A HYDROXYSTILBENE**
WÄSSRIGE KOSMETIKZUSAMMENSETZUNG MIT EINEM PYRIDINCARBONSÄUREDERIVAT UND EINEM HYDROXYSTILBEN
COMPOSITION COSMÉTIQUE AQUEUSE COMPRENANT UN DÉRIVÉ D'ACIDE PYRIDINEDICARBOXYLIQUE ET UN HYDROXYSTILBÈNE

(30) Priority: 17.07.2017 FR 1756724
(43) Date of publication of application: 27.05.2020
(73) Proprietor: L'OREAL, 75008 Paris (FR)
(72) Inventor: ISARD, Olivia, 93400 Saint Ouen (FR); TOURIGUINE, Olivia, 93400 Saint Ouen (FR); RICHET, Laurence, 93400 Saint Ouen (FR)
(74) Representative: Casalonga
(86) International application number: PCT/EP2018/069293
(87) International publication number: WO 2019/016153

(56) References cited:
- FR-A1- 3 030 248
- FR-A1- 3 044 549

## Description

The present invention relates to an aqueous cosmetic composition comprising at least one particular pyridinedicarboxylic acid derivative, or a salt thereof, and at least one hydroxystilbene, and also to the use thereof for caring for human keratin fibres such as the hair and the eyelashes, in particular for inducing and/or stimulating the growth and/or curbing the loss of said fibres as defined in claims.

The invention also relates to a process for cosmetic treatment of human keratin fibres using this composition.

The growth of the hair and its renewal are mainly determined by the activity of the hair follicles and their matrix environment. Their activity is cyclical and essentially comprises three phases, namely the anagenic phase, the catagenic phase and the telogenic phase.

The anagenic phase (active or growth phase), which lasts several years and during which the hairs lengthen, is followed by a very short and transient catagenic phase which lasts a few weeks, and then by a telogenic phase or resting phase which lasts a few months. At the end of the resting period, the hairs fall out and another cycle begins again. The head of hair is thus undergoing constant renewal and, of the approximately 150 000 hairs which make up a head of hair, approximately 10% are at rest and will be replaced in the months to come. The natural loss of the hair can be estimated, on average, at a few hundred hairs per day for a normal physiological state. This constant physical renewal process undergoes a natural change during the course of ageing; the hairs become finer and their cycles shorter. In addition, various causes may bring about substantial temporary or definitive hair loss. Hair loss, in particular alopecia, is essentially due to disruptions in hair renewal. These disruptions lead in a first stage to acceleration of the frequency of the cycles at the expense of the quality of the hairs, and then of their quantity. Progressive miniaturization of the bulbs takes place, in conjunction with isolation of these bulbs by gradual thickening of the perifollicular collagen matrix and also of the outer connective sheath. Revascularization around the hair follicle is thus made more difficult cycle after cycle. The hairs regress and become miniaturized until they are no more than an unpigmented down, and this phenomenon leads to gradual thinning of the head of hair. The number and mean diameter of the hair follicles that constitute the head of hair are affected. Certain areas are preferentially affected, especially the temporal or frontal lobes in men, and diffuse alopecia of the crown is observed in women.

The term "alopecia" also covers an entire family of hair follicle complaints of which the final consequence is partial or general definitive hair loss. It may more particularly be a case of androgenic alopecia. In a large number of cases, early hair loss occurs in genetically predisposed individuals, which is known as andro-chrono-genetic alopecia; this form of alopecia especially concerns men. It is known, furthermore, that certain factors, such as hormonal imbalance, physiological stress or malnutrition, can accentuate the phenomenon. In addition, loss or impairment of the hair can be in connection with seasonal phenomena.

Loss of hair density and hair loss are often experienced as distressing by persons thereby affected, especially when they are still young.

Pharmacological active agents such as minoxidil, latanoprost, fluridil, spironolactone and combinations thereof are known. However, they do not make it possible to maintain totally satisfactory hair density and may have adverse side effects.

Other products belonging to the cosmetic field exist. Among these, examples that may be mentioned include aminexil^{®} and stemoxydine^{®}.

However, consumers are still in search of more efficient products that have no adverse side effects, which would delay the process of "loss of hair density" or "excessive hair loss".

Furthermore, the products used for preventing loss of hair density often have cosmetic properties which are not very satisfactory. In particular, even in the case of an effective treatment, the head of hair visually lacks body and volume. This problem is all the greater in the case of fine hair.

Thus, there is a need to provide products which make it possible to effectively prevent and/or treat loss of hair density, while at the same time providing better cosmetic properties, in particular better properties in terms of styling the head of hair, and in particular products which make it possible to bring body and volume to the head of hair.

The applicant has found, surprisingly, that the combination, in an aqueous composition, of at least one pyridinedicarboxylic acid derivative of formula (I) as described below, or a salt thereof, with at least one hydroxystilbene, makes it possible to achieve these objectives.

A subject of the invention is therefore an aqueous cosmetic composition having a water content ranging from 25% to 60% by weight, and comprising:
- one or more pyridinedicarboxylic acid derivatives chosen from the compounds of general formula (I): 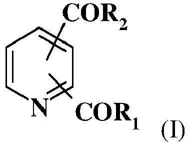 in which R₁ and R₂ each represent, independently of one another, a group -OR', R' representing a linear or branched, saturated or unsaturated C₁-C₁₈ alkyl group, and
- one or more hydroxystilbene(s) of the general formula (II) as defined in the claims.

The composition according to the invention makes it possible to obtain improved effects on the head of hair in particular regarding the hair density, the diameter or any other parameter representative of the quality of the head of hair, while at the same time providing the head of hair with better cosmetic properties. The composition according to the invention makes it possible in particular to give styling properties, and in particular body and volume to the head of hair.

The head of hair appears to be more voluminous and/or more robust, in particular in the case of fine hair.

The present invention also relates to a process for cosmetic treatment of human keratin fibres and/or of the scalp, comprising the application of the composition according to the invention to said fibres such as the hair and the eyelashes, and/or to the scalp.

A subject of the invention is also the use of the cosmetic composition for inducing and/or stimulating the growth of human keratin fibres such as the hair and the eyelashes, and/or for curbing their loss.

Other subjects, characteristics, aspects and advantages of the invention will emerge even more clearly on reading the description and the example that follows.

In the text hereinbelow, and unless otherwise indicated, the limits of a range of values are included within that range, especially in the expressions "between" and "ranging from ... to ...".

Moreover, the expression "at least one" used in the present description is equivalent to the expression "one or more".

The cosmetic composition according to the invention comprises one or more pyridinedicarboxylic acid derivatives which are compounds of general formula (I) or a salt thereof: 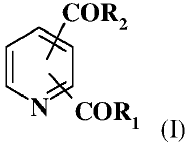 as defined above.

The C₁-C₁₈ alkyl group is preferably a saturated or unsaturated alkyl group comprising from 1 to 10 carbon atoms, such as methyl, ethyl, tert-butyl, isopropyl or hexyl. The alkyl group may contain at least one carbon-carbon double bond or carbon-carbon triple bond, for instance -CH=CH₂, -CH₂-CH=CH-CH₃ or -CH₂-C≡CH.

R' represents a C₁-C₁₈ and better still C₁-C₁₀ alkyl group.

In formula (I), R₁ and R₂ preferably represent, independently of one another, a group chosen from -OCH₃, -O-CH₂-CH₃, and -O-CH(CH₃)₂.

In one particular embodiment, -COR₁ and -COR₂ are, respectively, in positions 2 and 3, or in positions 2 and 4, of the pyridine nucleus. However, they may be in positions 2 and 5.

Unless otherwise mentioned in the present description, the use of the expression "pyridinedicarboxylic acid derivative" includes the derivative in non-salified form.

Examples of pyridinedicarboxylic acid derivatives that may especially be mentioned include:
- dimethyl 2,4-pyridinedicarboxylate,
- dimethyl 2,3-pyridinedicarboxylate,
- diethyl 2,4-pyridinedicarboxylate,
- diethyl 2,3-pyridinedicarboxylate,
- diisopropyl 2,4-pyridinedicarboxylate,
- diethyl 2,5-pyridinedicarboxylate,
- dimethyl 2,5-pyridinedicarboxylate.

The pyridinedicarboxylic acid derivative is in pyridinedicarboxylic acid ester form. The particularly preferred derivative is diethyl 2,4-pyridinedicarboxylate. This compound is sold, for example, under the trade name Stemoxydine^{®} by the company L'Oréal.

The composition according to the invention contains said derivative(s) in a content preferably ranging from 0.01% to 15% by weight, better still from 0.1% to 10% by weight, and even more preferentially from 1% to 8% by weight, relative to the total weight of the composition.

The hydroxystilbene(s) are chosen from the compounds of general formula (II): 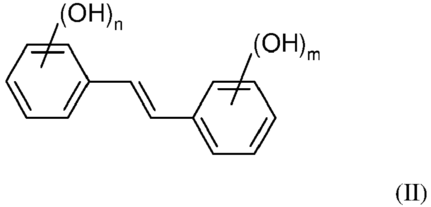 in which n is an integer ranging from 1 to 5, and m is an integer ranging from 0 to 5.

These compounds can be in a cis or trans form.

Hydroxystilbenes are compounds which are found in the natural state in the plants of the spermatophyte class and particularly in vines. Such compounds, for instance resveratrol, are found in grapes and wine.

In the prior art, hydroxystilbenes are used, inter alia, as depigmenting agents (JP87-192040), as vasodilators (EP 96-830517), as antithrombotic agents (JP 05016413), in the treatment of various cardiovascular conditions (CA 2187990), as mutagenesis-inhibiting and carcinogenesis-inhibiting agents (JP 06024967), or else are described as antioxidants.

Among these compounds, resveratrol (or 3,5,4'-trihydroxystilbene) is particularly studied for the above activities described mainly because it is a natural compound which is found in the skin of grape seeds and in wine. In this regard, the review by Soleas and colleagues (Clinical Biochemistry, vol. 30, n°2, pp. 91-113, 1997) may be consulted, said review perfectly summarizing the state of knowledge regarding this compound and hydroxystilbenes.

Among the hydroxystilbenes, mention may be made of mono-, di-, tri-, tetra-, penta-, hexa-, hepta-, octo- and nonahydroxystilbenes.

According to the invention, the hydroxystilbenes can be used alone or as mixtures of any nature and may be of natural or synthetic origin.

Preferably, in formula (II) n ranges from 1 to 3, better still n is equal to 1 or 2, and even better still n = 2; and m ranges from 0 to 3, better still m is equal to 1 or 2, and even better still m = 1.

The hydroxystilbenes preferentially used in the composition according to the invention are chosen from:
4'-hydroxy stilbene,
2',4'-dihydroxystilbene,
3',4'-dihydroxystilbene,
4,4'-dihydroxystilbene,
2',4',4-trihydroxystilbene,
3',4',4-trihydroxystilbene,
2,4,4'-trihydroxystilbene,
3,4,4'-trihydroxystilbene,
3,4',5-trihydroxystilbene,
2',3,4-trihydroxystilbene,
2,3',4-trihydroxystilbene,
2',2,4' -trihydroxy stilbene,
2,4,4',5-tetrahydroxystilbene,
2',3,4',5-tetrahydroxystilbene,
2,2',4,4'-tetrahydroxystilbene,
3,3',4',5-tetrahydroxystilbene,
2,3',4,4'-tetrahydroxystilbene,
3,3',4,4'-tetrahydroxystilbene,
3,3',4',5,5'-pentahydroxystilbene,
2,2',4,4',6-pentahydroxystilbene,
2,3',4,4',6-pentahydroxystilbene,
2,2',4,4',6,6'-hexahydroxystilbene,
and mixtures of these compounds.

Even more preferentially, the composition according to the invention contains 3,4',5-trihydroxystilbene or resveratrol.

The composition according to the invention contains said hydroxystilbene(s) in a content preferably ranging from 0.01% to 10% by weight, better still from 0.05% to 5% by weight, and more preferentially from 0.1% to 2% by weight, relative to the total weight of the composition.

The composition according to the invention is aqueous and comprises water in a content ranging from 25% to 60% by weight, relative to the total weight of the composition.

Preferably, the water content of the composition ranges from 30% to 55% by weight, relative to the total weight of said composition, better still from 30% to 50% by weight.

The composition according to the invention can also comprise one or more preferably water-soluble organic solvents that are liquid at 25°C and 1.013×10⁵ Pa.

Such organic solvents are preferentially chosen from alcohols comprising from 1 to 7 carbon atoms, and in particular C₁-C₇ aliphatic or aromatic monoalcohols, C₃-C₇ polyols and C₃-C₇ polyol ethers.

Particularly preferably, the composition comprises one or more organic solvents chosen from aliphatic monoalcohols comprising from 1 to 7 carbon atoms, preferentially comprising from 2 to 4 carbon atoms, in particular ethanol, isopropanol, and C₃-C₇ polyols, preferentially propylene glycol, and mixtures thereof. Better still, the composition comprises one or more organic solvents chosen from aliphatic monoalcohols comprising from 1 to 7 carbon atoms, preferentially comprising from 2 to 4 carbon atoms, more particularly ethanol, isopropanol, and mixtures thereof. Even better still, the composition according to the invention contains ethanol.

The composition according to the invention may comprise such organic solvent(s) in a content ranging from 5% to 70% by weight, preferably from 10% to 68% by weight, and more preferentially from 20% to 65% by weight, better still from 30% to 60% by weight, even better still from 40% to 60% by weight, relative to the total weight of the composition.

The composition according to the invention may also comprise one or more fixing polymer(s).

The term "fixing polymer" is intended to mean any polymer that is capable of giving a shape to a head of hair or of retaining a head of hair in a given shape.

The fixing polymer(s) used may be chosen from anionic, cationic, amphoteric and non-ionic fixing polymers, and mixtures thereof.

Anionic fixing polymers that may be mentioned include polymers comprising groups derived from carboxylic, sulfonic or phosphoric acids, and having a number-average molecular mass of between 500 and 5 000 000.

The carboxylic groups are provided by unsaturated monocarboxylic or dicarboxylic acid monomers, such as those corresponding to formula (I): 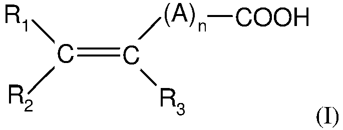 in which
n is an integer from 0 to 10,
A denotes a methylene group, optionally connected to the carbon atom of the unsaturated group or to the adjacent methylene group, when n is greater than 1, via a heteroatom, such as oxygen or sulfur,
R₁ denotes a hydrogen atom or a phenyl or benzyl group,
R₂ denotes a hydrogen atom, an alkyl group comprising from 1 to 4 carbon atoms or a carboxyl group,
R₃ denotes a hydrogen atom, an alkyl group comprising from 1 to 4 carbon atoms, a -CH₂-COOH group, a phenyl group or a benzyl group.

In formula (I) above, the alkyl group containing from 1 to 4 carbon atoms may in particular denote methyl and ethyl groups.

The anionic fixing polymers bearing carboxylic or sulfonic groups that are preferred are:
A) copolymers of acrylic or methacrylic acid or salts thereof, including copolymers of acrylic acid and acrylamide, and methacrylic acid/acrylic acid/ethyl acrylate/methyl methacrylate copolymers, more particularly Amerhold DR 25 sold by the company Amerchol, and sodium salts of polyhydroxycarboxylic acids. Mention may also be made of methacrylic acid/ethyl acrylate copolymers, in particular in aqueous dispersion, such as Luviflex Soft and Luvimer MAE, which are sold by the company BASF.
B) copolymers of acrylic or methacrylic acids with a monoethylenic monomer such as ethylene, styrene, vinyl esters and acrylic or methacrylic acid esters, optionally grafted onto a polyalkylene glycol such as polyethylene glycol and optionally crosslinked. Such polymers are described in particular in French patent 1 222 944 and German applicaton No. 2 330 956, the copolymers of this type comprising an optionally N-alkylated and/or hydroxyalkylated acrylamide unit in their chain as described especially in Luxembourg patent applications 75370 and 75371. Mention may also be made of copolymers of acrylic acid and C₁-C₄ alkyl methacrylate.

As another anionic fixing polymer from this class, mention may also be made of the butyl acrylate/acrylic acid/methacrylic acid branched block anionic polymer sold under the name Fixate G-100 L by the company Lubrizol (INCI name AMP-Acrylates/Allyl Methacrylate Copolymer).

C) copolymers derived from crotonic acid, such as those of which the chain comprises vinyl acetate or propionate units and optionally other monomers such as allyl or methallyl esters, vinyl ether or vinyl ester of a saturated, linear or branched carboxylic acid containing a long hydrocarbon-based chain such as those comprising at least 5 carbon atoms, it being possible for these polymers to be optionally grafted and crosslinked, or alternatively a vinyl, allyl or methallyl ester of an α- or β-cyclic carboxylic acid. Such polymers are described, inter alia, in French patents Nos. 1 222 944, 1 580 545, 2 265 782, 2 265 781, 1 564 110 and 2 439 798. Commercial products that fall within this category are the resins 282930, 261314 and 281310 sold by the company National Starch.

Mention may also be made, as copolymer derived from crotonic acid, of crotonic acid/vinyl acetate/vinyl tert-butylbenzoate terpolymers, and in particular Mexomer PW supplied by the company Chimex.

D) polymers derived from maleic, fumaric or itaconic acids or anhydrides with vinyl esters, vinyl ethers, vinyl halides, phenylvinyl derivatives or acrylic acid and esters thereof; these polymers may be esterified. Such polymers are described in particular in US patents 2047 398, 2 723 248 and 2 102 113 and GB patent 839 805, and especially those sold under the names Gantrez^{®} AN or ES by the company ISP.

Polymers also falling within this category are the copolymers of maleic, citraconic or itaconic anhydrides and of an allylic or methallylic ester optionally comprising an acrylamide or methacrylamide group, an α-olefin, acrylic or methacrylic esters, acrylic or methacrylic acids or vinylpyrrolidone in their chain, the anhydride functions being monoesterified or monoamidated. These polymers are described, for example, in French patents 2 350 384 and 2 357 241 by the Applicant.

E) polyacrylamides comprising carboxylate groups.

F) polymers comprising sulfonic groups. These polymers may be polymers comprising vinylsulfonic, styrenesulfonic, naphthalenesulfonic, acrylamidoalkylsulfonic or sulfoisophthalate units.

These polymers may be chosen especially from:
- polyvinylsulfonic acid salts with a molecular mass of between 1,000 and 100 000 approximately, and also the copolymers with an unsaturated comonomer such as acrylic or methacrylic acids and esters thereof, and also acrylamide or derivatives thereof, vinyl ethers and vinylpyrrolidone;
- polystyrenesulfonic acid salts and sodium salts, with a molecular mass of about 500 000 and of about 100 000. These compounds are described in patent FR 2198719;
- polyacrylamidesulfonic acid salts such as those mentioned in patent US 4 128 631;

G) grafted anionic silicone polymers.

The grafted silicone polymers used are preferably chosen from polymers containing a non-silicone organic backbone grafted with monomers containing a polysiloxane, polymers containing a polysiloxane backbone grafted with non-silicone organic monomers, and mixtures thereof.

H) anionic polyurethanes, possibly comprising silicone grafts and silicones containing hydrocarbon-based grafts.

Examples of fixing polyurethanes that may especially be mentioned include the dimethylolpropionic acid/isophorone diisocyanate/neopentyl glycol/polyester diols copolymer (also known under the name polyurethane-1, INCI name) sold under the brand name Luviset^{®} PUR by the company BASF, and the dimethylolpropionic acid/isophorone diisocyanate/neopentyl glycol/polyester diols/silicone diamine copolymer (also known under the name polyurethane-6, INCI name) sold under the brand name Luviset^{®} Si PUR A by the company BASF.

Another anionic polyurethane that may also be used is Avalure UR 450.

Polymers containing sulfoisophthalate groups, such as the polymers AQ55 and AQ48 sold by the company Eastman, may also be used.

According to the invention, the anionic fixing polymers are preferably chosen from acrylic acid copolymers, such as the acrylic acid/ethyl acrylate/N-tert-butylacrylamide terpolymer sold under the name Ultrahold Strong^{®} by the company BASF, methacrylic acid/ethyl acrylate copolymers, in particular in aqueous dispersion, such as Luviflex Soft and Luvimer MAE sold by the company BASF, crotonic acid-derived copolymers, such as the vinyl acetate/vinyl tert-butylbenzoate/crotonic acid terpolymers and the crotonic acid/vinyl acetate/vinyl neododecanoate terpolymers sold under the name Resin 28-29-30 by the company National Starch, polymers derived from maleic, fumaric or itaconic acids or anhydrides with vinyl esters, vinyl ethers, vinyl halides, phenylvinyl derivatives, acrylic acid and its esters, such as the monoesterified methyl vinyl ether/maleic anhydride copolymer sold under the name Gantrez^{®} ES 425 by the company ISP, Luviset Si PUR, Mexomer PW, elastomeric or non-elastomeric anionic polyurethanes, polymers comprising sulfoisophthalate groups, and anionic fixing polymers of the B) class; and even more particularly use is preferably made of the butyl acrylate/acrylic acid/methacrylic acid branched block anionic polymer sold under the name Fixate G-100 L by the company Lubrizol (INCI name AMP-Acrylates/Allyl Methacrylate Copolymer).

The cationic fixing polymers that may be used according to the present invention are preferably chosen from polymers comprising primary, secondary, tertiary and/or quaternary amine groups forming part of the polymer chain or directly attached thereto, and having a molecular weight of between 500 and approximately 5 000 000 and preferably between 1000 and 3 000 000.

Among these cationic fixing polymers, mention may be made more particularly of the following cationic polymers:
- quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in detail in French patents 2 077 143 and 2 393 573;
- dimethylaminoethyl methacrylate/vinylcaprolactam/vinylpyrrolidone terpolymers, such as the product sold under the name Gaffix VC 713 by the company ISP;
- quaternized vinylpyrrolidone/dimethylaminopropylmethacrylamide copolymers such as the product sold under the name Gafquat HS 100 by the company ISP;
- quaternary polymers of vinylpyrrolidone and of vinylimidazole, such as, for example, the products sold under the names Luviquat^{®} FC 905, FC 550 and FC 370 by the company BASF;
- vinylamine polymers comprising in their structure:
   (a) one or more units corresponding to formula (A) below: 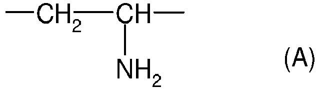
   (b) optionally one or more units corresponding to formula (B) below:

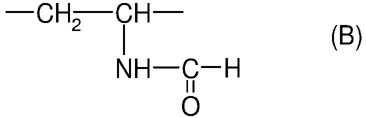

In other words, these polymers may be chosen in particular from homopolymers or copolymers comprising one or more units derived from vinylamine and optionally one or more units derived from vinylformamide.

Preferably, these cationic polymers are chosen from polymers comprising, in their structure, from 5 mol% to 100 mol% of units corresponding to the formula (A) and from 0 to 95 mol% of units corresponding to the formula (B), preferably from 10 mol% to 100 mol% of units corresponding to the formula (A) and from 0 to 90 mol% of units corresponding to the formula (B).

These polymers may be obtained, for example, by partial hydrolysis of polyvinylformamide. This hydrolysis may take place in acidic or basic medium.

The weight-average molecular weight of said polymer, measured by light scattering, may range from 1000 to 3 000 000 g/mol, preferably from 10 000 to 1000 000 and more particularly from 100 000 to 500 000 g/mol.

The polymers comprising units of formula (A) and optionally units of formula (B) are sold in particular under the Lupamin name by the company BASF, for instance, in a non-limiting way, the products provided under the names Lupamin 9095, Lupamin 5095, Lupamin 1095, Lupamin 9030 (or Luviquat 9030) and Lupamin 9010;
- polymers comprising a fatty chain and comprising a vinylpyrrolidone unit, such as the products sold under the names Styleze W20 and Styleze W10 by the company ISP;
- chitosans or salts thereof; the salts that may be used are in particular the acetate, lactate, glutamate, gluconate or pyrrolidonecarboxylate of chitosan.

Among these compounds, mention may be made of chitosan having a degree of deacetylation of 90.5% by weight, sold under the name Kytan Brut Standard by the company Aber Technologies, and chitosan pyrrolidonecarboxylate sold under the name Kytamer^{®} PC by the company Amerchol.

The amphoteric fixing polymers that can be used in accordance with the invention may be chosen from polymers comprising units B and C distributed randomly in the polymer chain, in which B denotes a unit deriving from a monomer comprising at least one basic nitrogen atom and C denotes a unit deriving from an acid monomer comprising one or more carboxylic or sulfonic groups, or alternatively B and C may denote groups deriving from carboxybetaine or sulfobetaine zwitterionic monomers; B and C can also denote a cationic polymer chain comprising primary, secondary, tertiary or quaternary amine groups, in which at least one of the amine groups bears a carboxylic or sulfonic group connected via a hydrocarbon-based group, or alternatively B and C form part of a chain of a polymer containing an ethylenedicarboxylic unit in which one of the carboxylic groups has been made to react with a polyamine comprising one or more primary or secondary amine groups.

The amphoteric fixing polymers corresponding to the definition given above that are more particularly preferred are chosen from the following polymers:
1) Polymers resulting from the copolymerization of a monomer derived from a vinyl compound bearing a carboxylic group, such as, more particularly, acrylic acid, methacrylic acid, maleic acid, α-chloroacrylic acid, and a basic monomer derived from a substituted vinyl compound containing at least one basic atom, such as, more particularly, dialkylaminoalkyl methacrylate and acrylate, dialkylaminoalkyl methacrylamide and acrylamide. Such compounds are described in patent US 3 836 537.
   The vinyl compound may also be a dialkyldiallylammonium salt such as diethyldiallylammonium chloride.
2) Polymers comprising units derived:
   a) from at least one monomer chosen from acrylamides and methacrylamides substituted on the nitrogen with an alkyl group,
   b) from at least one acidic comonomer containing one or more reactive carboxylic groups, and
   c) from at least one basic comonomer such as acrylic and methacrylic acid esters containing primary, secondary, tertiary and quaternary amine substituents, and the product of quaternization of dimethylaminoethyl methacrylate with dimethyl or diethyl sulfate.

The N-substituted acrylamides or methacrylamides that are more particularly preferred according to the invention are compounds in which the alkyl groups contain from 2 to 12 carbon atoms and more particularly N-ethylacrylamide, N-tert-butylacrylamide, N-tert-octylacrylamide, N-octylacrylamide, N-decylacrylamide and N-dodecylacrylamide, and the corresponding methacrylamides.

The acidic comonomers are more particularly chosen from acrylic acid, methacrylic acid, crotonic acid, itaconic acid, maleic acid and fumaric acid and alkyl monoesters, containing 1 to 4 carbon atoms, of maleic or fumaric acids or anhydrides. The preferred basic comonomers are aminoethyl, butylaminoethyl, N,N'-dimethylaminoethyl and N-tert-butylaminoethyl methacrylates. The copolymers of which the CTFA (4th edition, 1991) name is octylacrylamide/acrylates/butylaminoethyl methacrylate copolymer, such as the products sold under the name Amphomer^{®} or Lovocryl^{®} 47 by the company National Starch, are particularly used.

3) Crosslinked and alkylated polyaminoamides partially or totally derived from polyaminoamides of general formula (II): 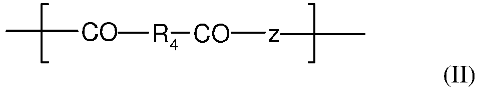 in which R₄ represents a divalent group derived from a saturated dicarboxylic acid, from a mono- or dicarboxylic aliphatic acid with an ethylenic double bond, from an ester of an alcohol containing 1 to 6 carbon atoms with these acids, or from a group deriving from the addition of any one of said acids with a bis-primary amine or bis-secondary-derived amine, and Z denotes a group of a bis-primary or mono- or bis-secondary polyalkylene-polyamine, and preferably represents:
a) in proportions of from 60 mol% to 100 mol%, the group (III) 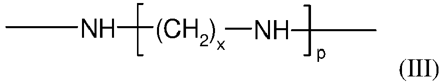
   in which x = 2 and p = 2 or 3, or else x = 3 and p = 2,
   this group being derived from diethylenetriamine, triethylenetetramine or dipropylenetriamine;
b) in proportions of 0 to 40 mol%, the group (IV) above, in which x = 2 and p = 1, which derives from ethylenediamine, or the group deriving from piperazine; 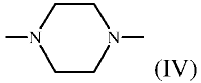
c) in proportions of from 0 to 20 mol%, the group -NH-(CH₂)₆-NH- deriving from hexamethylenediamine, these polyaminoamines being crosslinked by addition of a difunctional crosslinking agent chosen from epihalohydrins, diepoxides, dianhydrides, bis-unsaturated derivatives, by means of 0.025 to 0.35 mol of crosslinking agent per amine group of the polyaminoamide, and being alkylated by the action of acrylic acid, chloroacetic acid or an alkane sultone or salts thereof.

The saturated carboxylic acids are preferably chosen from acids containing 6 to 10 carbon atoms, such as adipic acid, 2,2,4-trimethyladipic acid, 2,4,4-trimethyladipic acid and terephthalic acid, and acids bearing an ethylenic double bond, for instance acrylic, methacrylic and itaconic acids. The alkane sultones used in the alkylation are preferably propane sultone or butane sultone, the salts of the alkylating agents are preferably the sodium or potassium salts.

(4) Polymers comprising zwitterionic units of formula (V): 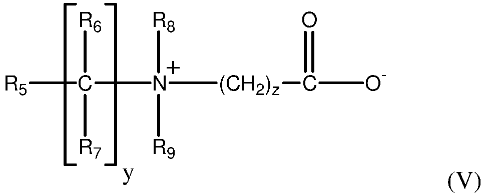 in which
R₅ denotes a polymerizable unsaturated group such as an acrylate, methacrylate, acrylamide or methacrylamide group,
y and z each represent an integer from 1 to 3,
R₆ and R₇ represent a hydrogen atom or a methyl, ethyl or propyl group,
R₈ and R₉ represent a hydrogen atom or an alkyl group such that the sum of the carbon atoms in R₁₀ and R₁₁ does not exceed 10.

The polymers comprising such units may also comprise units derived from non-zwitterionic monomers such as dimethyl- or diethylaminoethyl acrylate or methacrylate or alkyl acrylates or methacrylates, acrylamides or methacrylamides or vinyl acetate.

5) Polymers derived from chitosan comprising monomer units corresponding to the following formulae (VI), (VII) and (VIII) below: 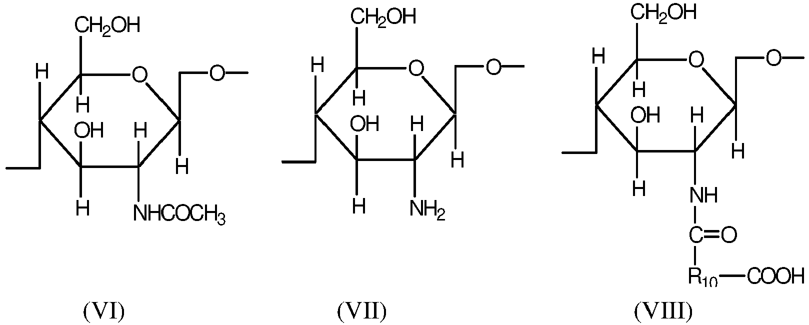 the unit (VI) being present in proportions of between 0 and 30%, the unit (VII) in proportions of between 5% and 50% and the unit (VIII) in proportions of between 30% and 90%, it being understood that, in this unit (VIII), R₁₀ represents a group of formula (IX): 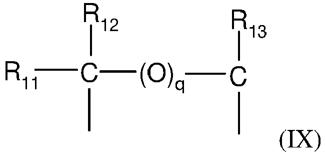 in which, if q = 0, R₁₁, R₁₂ and R₁₃, which may be identical or different, each represent a hydrogen atom, a methyl, hydroxyl, acetoxy or amino residue, a monoalkylamine residue or a dialkylamine residue that are optionally interrupted with one or more nitrogen atoms and/or optionally substituted with one or more amine, hydroxyl, carboxyl, alkylthio or sulfonic groups, an alkylthio residue in which the alkyl group bears an amino residue, at least one of the groups R₁₁, R₁₂ and R₁₃ being, in this case, a hydrogen atom;
or, if q = 1, R₁₁, R₁₂ and R₁₃ each represent a hydrogen atom, and also the salts formed by these compounds with bases or acids.

6) Polymers derived from the N-carboxyalkylation of chitosan.

7) Polymers of units corresponding to the general formula (X) described, for example, in French patent 1 400 366: 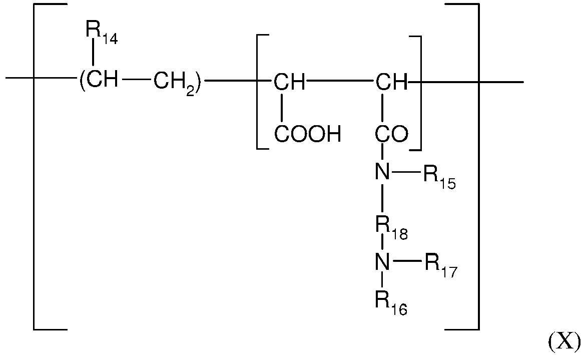 in which R₁₄ represents a hydrogen atom or a CH₃O, CH₃CH₂O, or phenyl group, R₁₅ denotes hydrogen or a C₁-C₄ alkyl group such as methyl and ethyl, R₁₆ denotes hydrogen or a C₁-C₄ alkyl group such as methyl and ethyl, R₁₇ denotes a C₁-C₄ alkyl group such as methyl and ethyl or a group corresponding to the formula: -R₁₈-N(R₁₆)₂, with R₁₈ representing a -CH₂-CH₂-, -CH₂-CH₂-CH₂-, or -CH₂-CH(CH₃)-group and R₁₆ having the meanings given above,
and also the higher homologues of these groups, containing up to 6 carbon atoms.

8) Amphoteric polymers of the type -D-X-D-X-, chosen from: a) polymers obtained by the action of chloroacetic acid or sodium chloroacetate on compounds comprising at least one unit of formula:

-D-X-D-X-D- (XI)

in which D denotes a group 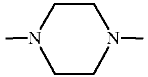 and X denotes the symbol E or E', where E or E', which may be identical or different, denote a divalent group that is an alkylene group with a straight or branched chain containing up to 7 carbon atoms in the main chain, which is unsubstituted or substituted with hydroxyl groups and which can comprise, in addition to oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups.

b) polymers of formula:

-D-X-D-X- (XII)

in which D denotes a group 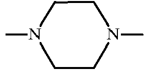 and X denotes the symbol E or E' and at least once E'; E having the meaning given above and E' is a divalent group that is an alkylene group with a straight or branched chain containing up to 7 carbon atoms in the main chain, which is unsubstituted or substituted with one or more hydroxyl groups and which contains one or more nitrogen atoms, the nitrogen atom being substituted with an alkyl chain that is optionally interrupted with an oxygen atom and necessarily comprising one or more carboxyl functions or one or more hydroxyl functions, betainized by reaction with chloroacetic acid or sodium chloroacetate.

9) (C₁-C₅)Alkyl vinyl ether/maleic anhydride copolymers partially modified by semiamidation with an N,N-dialkylaminoalkylamine such as N,N-dimethylaminopropylamine or by semiesterification with an N,N-dialkanolamine. These copolymers may also comprise other vinyl comonomers such as vinylcaprolactam.

According to a preferred embodiment, the amphoteric fixing polymers that may be used in the invention may be chosen from branched block copolymers comprising:
(a) non-ionic units derived from at least one monomer chosen from C₁-C₂₀ alkyl (meth)acrylates, N-mono-(C₂-C₁₂ alkyl)(meth)acrylamides and N,N-di(C₂-C₁₂ alkyl)(meth)acrylamides,
(b) anionic units derived from at least one monomer chosen from acrylic acid and methacrylic acid, and
(c) polyfunctional units derived from at least one monomer comprising at least two polymerizable unsaturated functional groups,
and preferably having a structure constituted of hydrophobic blocks onto which are fixed, via polyfunctional units (c), several blocks that are more hydrophilic.

Preferably, the amphoteric polymers have at least two glass transition temperatures (Tg), at least one of which is greater than 20°C and the other of which is less than 20°C.

The preferred amphoteric polymers are polymers comprising units derived:
a) from at least one monomer chosen from acrylamides and methacrylamides substituted on the nitrogen with an alkyl group,
b) from at least one acidic comonomer containing one or more reactive carboxylic groups, and
c) from at least one basic comonomer such as acrylic and methacrylic acid esters bearing primary, secondary, tertiary and quaternary amine substituents, and the product of quaternization of dimethylaminoethyl methacrylate with dimethyl or diethyl sulfate.

Mention may be made in particular of the polymers sold under the name Amphomer by the company National Starch.

The non-ionic fixing polymers that may be used according to the present invention are chosen, for example, from:
- polyalkyloxazolines;
- vinyl acetate homopolymers,
- vinyl acetate copolymers, for instance copolymers of vinyl acetate and of acrylic ester, copolymers of vinyl acetate and of ethylene, or copolymers of vinyl acetate and of maleic ester, for example of dibutyl maleate,
- acrylic ester homopolymers and copolymers, for instance copolymers of alkyl acrylates and of alkyl methacrylates, such as the products sold by the company Röhm & Haas under the names Primal^{®} AC-261 K and Eudragit^{®} NE 30 D, by the company BASF under the name 8845, or by the company Hoechst under the name Appretan^{®} N9212,
- copolymers of acrylonitrile and of a non-ionic monomer chosen, for example, from butadiene and alkyl (meth)acrylates, such as the products provided under the name CJ 0601 B by the company Röhm & Haas,
- styrene homopolymers,
- styrene copolymers, for instance copolymers of styrene and of alkyl (meth)acrylate, such as the products Mowilith^{®} LDM 6911, Mowilith^{®} DM 611 and Mowilith^{®} LDM 6070 sold by the company Hoechst, and the products Rhodopas^{®} SD 215 and Rhodopas^{®} DS 910 sold by the company Rhône-Poulenc; copolymers of styrene, of alkyl methacrylate and of alkyl acrylate; copolymers of styrene and of butadiene; or copolymers of styrene, of butadiene and of vinylpyridine;
- polyamides,
- vinyllactam homopolymers such as vinylpyrrolidone homopolymers and the polyvinylcaprolactam sold under the name Luviskol^{®} Plus by the company BASF,
- vinyllactam copolymers such as a poly(vinylpyrrolidone/vinyllactam) copolymer sold under the trade name Luvitec^{®} VPC 55K65W by the company BASF, poly(vinylpyrrolidone/vinyl acetate) copolymers, such as those sold under the name PVPVA^{®} S630L by the company ISP, Luviskol^{®} VA 73, VA 64, VA 55, VA 37 and VA 28 by the company BASF; and poly(vinylpyrrolidone/vinyl acetate/vinyl propionate) terpolymers, for instance the product sold under the name Luviskol^{®} VAP 343 by the company BASF, and
- poly(vinyl alcohols).

The alkyl groups of the non-ionic polymers mentioned above preferably contain from 1 to 6 carbon atoms.

Preferably, the fixing polymer(s) are chosen from non-ionic fixing polymers and anionic fixing polymers, and mixtures thereof. More preferentially, the fixing polymer(s) are chosen from anionic fixing polymers such as those described above, and even more preferentially from polymers derived from maleic, fumaric or itaconic acids or anhydrides with vinyl esters, vinyl ethers, vinyl halides, phenylvinyl derivatives, acrylic acid and esters thereof such as the monoesterified methyl vinyl ether/maleic anhydride copolymers sold under the names Gantrez^{®} ES 225 and Gantrez^{®} ES 425 by the company ISP, and blends of such polymers.

The content of the fixing polymer(s) in the composition according to the invention can range from 0.01% to 10% by weight, preferably from 0.05% to 5% by weight, and more preferentially from 0.1% to 2% by weight, better still from 0.2% to 1% by weight, relative to the total weight of the composition.

The composition according to the invention may also comprise one or more additional compounds chosen in particular from surfactants, preferably chosen from non-ionic, anionic, cationic and amphoteric surfactants, conditioning agents preferably chosen from cationic polymers, silicones, thickening agents, which are polymeric or non-polymeric, and natural or synthetic, UV filters, fillers such as nacres, titanium dioxide, resins and clays, fragrances, peptizers, vitamins, preservatives, acidic agents, alkaline agents, reducing agents, oxidizing agents, and a mixture of these compounds.

The above additional compounds may each be present in an amount ranging from 0% to 20% by weight relative to the total weight of the composition.

The composition according to the invention is intended for cosmetic use by topical application to the skin and/or keratin fibres, and more especially to the scalp, the head of hair and the eyelashes.

According to the application method, this composition may be in any galenical form normally used in cosmetics, such as a lotion, serum, milk, cream, gel, ointment, pomade, powder, balm, patch, impregnated pad, soap, bar or foam. It may be formulated in the form of an emulsion, such as an oil-in-water direct emulsion or a water-in-oil inverse emulsion.

For topical application to the skin, including the scalp, the composition may especially be in the form of an aqueous or aqueous-alcoholic solution or suspension, an emulsion or dispersion of liquid or semi-liquid consistency obtained by dispersing a fatty phase in an aqueous phase (O/W) or vice versa (W/O), a dispersion or emulsion of soft consistency, an aqueous or aqueous-alcoholic gel, or else microcapsules or microparticles, or vesicular dispersions of ionic and/or non-ionic type.

The composition may also be in the form of a foam or else in the form of an aerosol composition also comprising a pressurized propellant.

In particular, when it is intended to be applied to be scalp or the hair, the composition can be in the form of a hair lotion, shampoo, conditioner, hair shaping product (lacquer, hair setting product, styling gel), a hair mask, or a foaming cream or gel for cleansing the hair.

According to one preferred embodiment, the composition according to the invention is in the form of a cream or lotion, a shampoo or conditioner.

The present invention also relates to a process for cosmetic treatment of human keratin fibres and/or of the scalp, comprising the application of the composition described above to the fibres and/or the scalp, and more particularly to the hair, the eyelashes and/or the scalp.

In particular the process comprises applying to the scalp and/or the fibres the composition according to the invention, then, optionally, rinsing, after a composition leave-on time which can range from 1 minute to 30 minutes.

Preferably, the cosmetic composition according to the invention is not rinsed off.

Finally, a subject of the present invention is a composition as described above, for use thereof for inducing and/or stimulating the growth of human keratin fibres such as the hair and the eyelashes, and/or for curbing the loss thereof.

The examples that follow serve to illustrate the invention.

### EXAMPLES

### Example 1:

The following compositions were prepared from the ingredients indicated in the table below, in which all the amounts are indicated as weight of active material, relative to the total weight of the composition.

| Ingredients | Comparative composition A | Composition B of the invention |
|---|---|---|
| Resveratrol | - | 0.25 |
| Methyl vinyl ether / ethyl monomaleate copolymer in solution at 50% in ethanol (1) | 0.25 | 0.25 |
| Tocopheryl acetate | 0.01 | 0.01 |
| Diethyl 2,4-pyridinedicarboxylate | 5 | 5 |
| Fragrance | 0.4 | 0.4 |
| Ethanol | 56.5 | 56.5 |
| Water | qs 100 | qs 100 |

### (1) sold under the name Gantrez ES 225 by ISP (Ashland)

Compositions A and B were applied as a single application to locks of natural hair weighing 2.7 g, washed beforehand with a neutral shampoo. The amount applied is 0.40 g of composition per lock. The locks were not rinsed after application of each composition.

Experts compared the cosmetic performance levels obtained by application of compositions A and B above.

Composition B according to the invention provides better styling properties than comparative composition A. The head of hair shows more body and robustness to the feel, and more volume visually.

### Example 2:

The following compositions were prepared from the ingredients indicated in the table below, in which all the amounts are indicated as weight of active material, relative to the total weight of the composition.

| Ingredients | Comparative composition C | Composition D of the invention |
|---|---|---|
| Diethyl 2,4-pyridinedicarboxylate (1) | 4 | 4 |
| Resveratrol | 0.2 | 0.2 |
| Propylene glycol | 10 | 10 |
| Water | - | 25 |
| Isopropyl alcohol | qs 100 | qs 100 |

### (1) sold under the name Diethyllutidinate by Mexoryl SBU

Compositions C and D were applied as a single application to locks of natural hair weighing 2.7 g, washed beforehand with a neutral shampoo. The amount applied is 0.15 g of composition per lock. The locks were not rinsed after application of each composition. The locks were then dried.

The performance levels in terms of smooth and soft feel were evaluated by 5 experts, on the locks of dry hair, by means of a blind test. Each of the 5 experts denoted the lock which had the smoothest and softest feel, when sliding the hair between the fingers over the entire length of the lock.

The 5 experts also evaluated the sheen of the locks.

The results obtained are detailed in the table below:

| | Smoothness/softness | | Sheen | |
|---|---|---|---|---|
| | C comparative | D invention | C comparative | D invention |
| Expert 1 | - | + | - | + |
| Expert 2 | - | + | + | - |
| Expert 3 | - | + | - | + |
| Expert 4 | - | + | - | + |
| Expert 5 | - | + | - | + |
| % in favour of D | 100% | | 80% | |

5 experts out of 5 (100%) judged that the fibres treated with composition (D) according to the invention were smoother and softer than those treated with comparative composition (C).

4 experts out of 5 (80%) judged that the fibres treated with composition (D) according to the invention were shinier than those treated with comparative composition (C).

Compared with composition C, composition D according to the invention results in better performance levels in terms of smooth and soft feel and also in terms of sheen.

## Claims

1. Aqueous cosmetic composition having a water content ranging from 25% to 60% by weight, and comprising:
• one or more pyridinedicarboxylic acid derivative(s) chosen from the compounds of general formula (I): 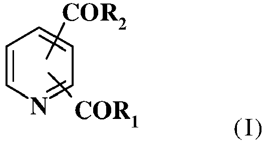
in which R₁ and R₂ each represent, independently of one another, a group -OR',
R' representing a linear or branched, saturated or unsaturated C₁-C₁₈ alkyl group, and
• one or more hydroxystilbene(s) chosen from the compounds of general formula (II): 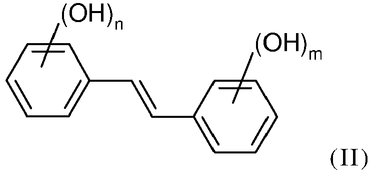 in which n is an integer ranging from 1 to 5, and m is an integer ranging from 0 to 5.

2. Composition according to Claim 1, **characterized in that** R₁ and R₂ represent, independently of one another, a group chosen from, - OCH₃, -O-CH₂-CH₃ and -O-CH(CH₃)₂.

3. Composition according to Claim 1 or 2, **characterized in that** -COR₁ and -COR₂ are, respectively, in positions 2 and 3, or in positions 2 and 4, of the pyridine nucleus.

4. Composition according to any one of the preceding claims, **characterized in that** the pyridinedicarboxylic acid derivative is chosen from:
- dimethyl 2,4-pyridinedicarboxylate,
- dimethyl 2,3-pyridinedicarboxylate,
- diethyl 2,4-pyridinedicarboxylate,
- diethyl 2,3-pyridinedicarboxylate,
- diisopropyl 2,4-pyridinedicarboxylate,
- diethyl 2,5-pyridinedicarboxylate,
- dimethyl 2,5-pyridinedicarboxylate,
and preferably the pyridinedicarboxylic acid derivative is diethyl 2,4-pyridinedicarboxylate.

5. Composition according to one of the preceding claims, **characterized in that** it contains said pyridinedicarboxylic acid derivative(s) in a content ranging from 0.01% to 15% by weight, preferably from 0.1% to 10% by weight, and more preferentially from 1% to 8% by weight, relative to the total weight of the composition.

6. Composition according to any one of the preceding claims, **characterized in that** the hydroxystilbene(s) is (are) chosen from mono-, di-, tri-, tetra-, penta-, hexa-, hepta-, octo-, and nonahydroxystilbenes, and preferably from:
4'-hydroxystilbene,
2',4'-dihydroxystilbene,
3',4'-dihydroxystilbene,
4,4'-dihydroxystilbene,
2',4',4-trihydroxystilbene,
3',4',4-trihydroxystilbene,
2,4,4'-trihydroxystilbene,
3,4,4'-trihydroxystilbene,
3,4',5-trihydroxystilbene,
2',3,4-trihydroxystilbene,
2,3',4-trihydroxy stilbene,
2',2,4'-trihydroxystilbene,
2,4,4',5-tetrahydroxystilbene,
2',3,4',5-tetrahydroxystilbene,
2,2',4,4'-tetrahydroxystilbene,
3,3',4',5-tetrahydroxystilbene,
2,3',4,4'-tetrahydroxy stilbene,
3,3',4,4'-tetrahydroxy stilbene,
3,3',4',5,5'-pentahydroxystilbene,
2,2',4,4',6-pentahydroxystilbene,
2,3',4,4',6-pentahydroxystilbene,
2,2',4,4',6,6'-hexahydroxystilbene,
and mixtures of these compounds,
and more preferentially, the composition contains 3,4',5-trihydroxy stilbene.

7. Composition according to any one of the preceding claims, **characterized in that** it contains said hydroxystilbene(s) in a content ranging from 0.01% to 10% by weight, preferably from 0.05% to 5% by weight, more preferentially from 0.1% to 2% by weight, relative to the total weight of the composition.

8. Composition according to any one of the preceding claims, **characterized in that** its water content ranges from 30% to 55% by weight, relative to the total weight of the composition, and preferably from 30% to 50% by weight.

9. Composition according to any one of the preceding claims, **characterized in that** it also comprises one or more organic solvents chosen from alcohols comprising from 1 to 7 carbon atoms; preferably from C₁-C₇ aliphatic or aromatic monoalcohols, and C₃-C₇ polyols and polyol ethers; better still chosen from aliphatic monoalcohols comprising from 1 to 7 carbon atoms, more preferentially comprising from 2 to 4 carbon atoms.

10. Composition according to the preceding claim, **characterized in that** it comprises the organic solvent(s) in a content ranging from 5% to 70% by weight, preferably from 10% to 68% by weight, more preferentially from 20% to 65% by weight, and better still from 30% to 60% by weight, relative to the total weight of the composition.

11. Composition according to any one of the preceding claims, **characterized in that** it also comprises one or more fixing polymer(s), preferably chosen from non-ionic fixing polymers and anionic fixing polymers, better still from anionic fixing polymers, in a content ranging from 0.01% to 10% by weight, preferably from 0.05% to 5% by weight, and more preferentially from 0.1% to 2% by weight, better still from 0.2% to 1% by weight, relative to the total weight of the composition.

12. Non-therapeutic cosmetic process of treatment of human keratin fibres and/or of the scalp, comprising the application to said fibres and/or the scalp of a composition as defined in any one of the preceding claims for inducing and/or stimulating the growth of human keratin fibres such as the hair and the eyelashes, and/or for curbing their loss.

13. Non-therapeutic cosmetic use of the composition according to any one of Claims 1 to 11, for inducing and/or stimulating the growth of human keratin fibres such as the hair and the eyelashes, and/or for curbing their loss.

## Patentansprüche

1. Wässrige kosmetische Zusammensetzung mit einem Wassergehalt im Bereich von 25 bis 60 Gew.-% und umfassend:
• ein oder mehrere Pyridindicarbonsäurederivate, ausgewählt aus den Verbindungen der allgemeinen Formel (I): 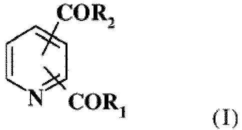
in der R₁ und R₂ jeweils unabhängig voneinander für eine Gruppe -OR' stehen,
wobei R' für eine lineare oder verzweigte, gesättigte oder ungesättigte C₁-C₁₈-Alkylgruppe steht, und
• ein oder mehrere Hydroxystilbene, ausgewählt aus den Verbindungen der allgemeinen Formel (II): 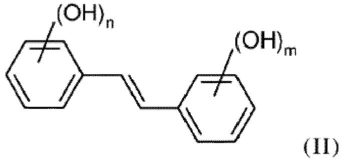 in der n für eine ganze Zahl im Bereich von 1 bis 5 steht und m für eine ganze Zahl im Bereich von 0 bis 5 steht.

2. Zusammensetzung nach Anspruch 1, **dadurch gekennzeichnet, dass** R₁ und R₂ jeweils unabhängig voneinander für eine aus -OCH₃, -O-CH₂-CH₃ und -O-CH(CH₃)₂ ausgewählte Gruppe stehen.

3. Zusammensetzung nach Anspruch 1 oder 2, **dadurch gekennzeichnet, dass** -COR₁ und -COR₂ jeweils in den Positionen 2 und 3 oder in den Positionen 2 und 4 des Pyridinkerns stehen.

4. Zusammensetzung nach einem der vorhergehenden Ansprüche, **dadurch gekennzeichnet, dass** das Pyridindicarbonsäurederivat ausgewählt ist aus:
- 2,4-Pyridindicarbonsäuredimethylester,
- 2,3-Pyridindicarbonsäuredimethylester,
- 2,4-Pyridindicarbonsäurediethylester,
- 2,3-Pyridindicarbonsäurediethylester,
- 2,4-Pyridindicarbonsäurediisopropylester,
- 2,5-Pyridindicarbonsäurediethylester,
- 2,5-Pyridindicarbonsäuredimethylester,
und es sich bei dem Pyridindicarbonsäurederivat vorzugsweise um 2,4-Pyridindicarbonsäurediethylester handelt.

5. Zusammensetzung nach einem der vorhergehenden Ansprüche, **dadurch gekennzeichnet, dass** sie das Pyridindicarbonsäurederivat bzw. die Pyridindicarbonsäurederivate in einem Gehalt im Bereich von 0,01 bis 15 Gew.-%, vorzugsweise von 0,1 bis 10 Gew.-% und weiter bevorzugt von 1 bis 8 Gew.-%, bezogen auf das Gesamtgewicht der Zusammensetzung, enthält.

6. Zusammensetzung nach einem der vorhergehenden Ansprüche, **dadurch gekennzeichnet, dass** das Hydroxystilben bzw. die Hydroxystilbene aus Mono-, Di-, Tri-, Tetra-, Penta-, Hexa-, Hepta-, Octo- und Nonahydroxystilbenen und vorzugsweise aus
4'-Hydroxystilben,
2',4'-Dihydroxystilben,
3',4'-Dihydroxystilben,
4,4'-Dihydroxystilben,
2',4',4-Trihydroxystilben,
3',4',4-Trihydroxystilben,
2,4,4'-Trihydroxystilben,
3,4,4'-Trihydroxystilben,
3,4',5-Trihydroxystilben,
2',3,4-Trihydroxystilben,
2,3',4-Trihydroxystilben,
2',2,4'-Trihydroxystilben,
2,4,4',5-Tetrahydroxystilben,
2',3,4',5-Tetrahydroxystilben,
2,2',4,4'-Tetrahydroxystilben,
3,3',4',5-Tetrahydroxystilben,
2,3',4,4'-Tetrahydroxystilben,
3,3',4,4'-Tetrahydroxystilben,
3,3',4',5,5'-Pentahydroxystilben,
2,2',4,4',6-Pentahydroxystilben,
2,3',4,4',6-Pentahydroxystilben,
2,2',4,4',6,6'-Hexahydroxystilben
und Mischungen dieser Verbindungen
ausgewählt ist bzw. sind und die Zusammensetzung weiter bevorzugt 3,4',5-Trihydroxystilben enthält.

7. Zusammensetzung nach einem der vorhergehenden Ansprüche, **dadurch gekennzeichnet, dass** sie das Hydroxystilben bzw. die Hydroxystilbene in einem Gehalt im Bereich von 0,01 bis 10 Gew.-%, vorzugsweise von 0,05 bis 5 Gew.-%, weiter bevorzugt von 0,1 bis 2 Gew.-%, bezogen auf das Gesamtgewicht der Zusammensetzung, enthält.

8. Zusammensetzung nach einem der vorhergehenden Ansprüche, **dadurch gekennzeichnet, dass** ihr Wassergehalt im Bereich von 30 bis 55 Gew.-%, bezogen auf das Gesamtgewicht der Zusammensetzung, und vorzugsweise 30 bis 50 Gew.-% beträgt.

9. Zusammensetzung nach einem der vorhergehenden Ansprüche, **dadurch gekennzeichnet, dass** sie außerdem ein oder mehrere organische Lösungsmittel umfasst, die aus Alkoholen mit 1 bis 7 Kohlenstoffatomen, vorzugsweise aus aliphatischen oder aromatischen C₁-C₇-Monoalkoholen und C₃-C₇-Polyolen und -Polyolethern, ausgewählt sind und noch besser aus aliphatischen Monoalkoholen mit 1 bis 7 Kohlenstoffatomen und weiter bevorzugt mit 2 bis 4 Kohlenstoffatomen ausgewählt sind.

10. Zusammensetzung nach dem vorhergehenden Anspruch, **dadurch gekennzeichnet, dass** sie das organische Lösungsmittel bzw. die organischen Lösungsmittel in einem Gehalt im Bereich von 5 bis 70 Gew.-%, vorzugsweise von 10 bis 68 Gew.-%, weiter bevorzugt von 20 bis 65 Gew.-% und noch besser von 30 bis 60 Gew.-%, bezogen auf das Gesamtgewicht der Zusammensetzung, umfasst.

11. Zusammensetzung nach einem der vorhergehenden Ansprüche, **dadurch gekennzeichnet, dass** sie außerdem ein oder mehrere fixierende Polymere, die vorzugsweise aus nichtionischen fixierenden Polymeren und anionischen fixierenden Polymeren und noch besser aus anionischen fixierenden Polymeren ausgewählt sind, in einem Gehalt im Bereich von 0,01 bis 10 Gew.-%, vorzugsweise von 0,05 bis 5 Gew.-%, weiter bevorzugt von 0,1 bis 2 Gew.-% und noch besser von 0,2 bis 1 Gew.-%, bezogen auf das Gesamtgewicht der Zusammensetzung, umfasst.

12. Nichttherapeutisches kosmetisches Verfahren zur Behandlung von menschlichen Keratinfasern und/oder der Kopfhaut, umfassend das Aufbringen einer Zusammensetzung gemäß einem der vorhergehenden Ansprüche auf die Fasern und/oder die Kopfhaut zur Induzierung und/oder Stimulierung des Wachstums von menschlichen Keratinfasern wie dem Haar und den Wimpern und/oder zur Eindämmung ihres Ausfallens.

13. Nichttherapeutische kosmetische Verwendung der Zusammensetzung nach einem der Ansprüche 1 bis 11 zur Induzierung und/oder Stimulierung des Wachstums von menschlichen Keratinfasern wie dem Haar und den Wimpern und/oder zur Eindämmung ihres Ausfallens.

## Revendications

1. Composition cosmétique aqueuse ayant une teneur en eau allant de 25 à 60% en poids, et comprenant :
• un ou plusieurs dérivés d'acide pyridine-dicarboxylique de formule générale (I): 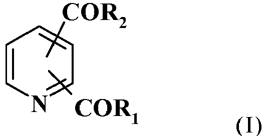
dans laquelle R₁ et R₂ représentent chacun, indépendamment l'un de l'autre, un groupe -OR',
R' représentant un groupe alkyle en C₁-C₁₈, linéaire ou ramifié, saturé ou insaturé, et
• un ou plusieurs hydroxystilbène(s) choisis parmi les composés de formule générale (II) : 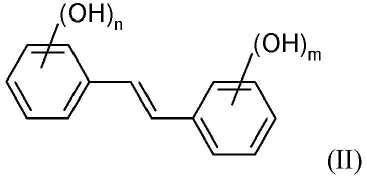 dans laquelle n est un nombre entier allant de 1 à 5 et m est un nombre entier allant de 0 à 5.

2. Composition selon la revendication 1, **caractérisée en ce que** R₁ et R₂ représentent indépendamment l'un de l'autre, un groupe choisi parmi -OCH₃, -O-CH₂-CH₃ et -O-CH(CH₃)₂.

3. Composition selon la revendication 1 ou 2, **caractérisée en ce que** -COR₁ et -COR₂ sont respectivement en positions 2 et 3, ou en positions 2 et 4 du noyau pyridine.

4. Composition selon l'une quelconque des revendications précédentes, **caractérisée** en que le dérivé d'acide pyridine-dicarboxylique est choisi parmi :
- le pyridine-2,4-dicarboxylate de diméthyle,
- le pyridine-2,3-dicarboxylate de diméthyle,
- le pyridine-2,4-dicarboxylate de diéthyle,
- le pyridine-2,3-dicarboxylate de diéthyle,
- le pyridine-2,4-dicarboxylate de di-isopropyle,
- le pyridine-2,5-dicarboxylate de diéthyle,
- le pyridine-2,5-dicarboxylate de diméthyle,
et de préférence le dérivé d'acide pyridine-dicarboxylique est le pyridine-2,4 dicarboxylate de diéthyle.

5. Composition selon l'une des revendications précédentes, **caractérisée en ce qu'**elle contient ledit (lesdits) dérivé(s) d'acide pyridine-dicarboxylique en une teneur allant de 0,01 à 15 % en poids, de préférence de 0,1 à 10 % en poids, et plus préférentiellement de 1 à 8 % en poids, par rapport au poids total de la composition.

6. Composition selon l'une quelconque des revendications précédentes, **caractérisée en ce que** le (les) hydroxystilbène(s) est (sont) choisis parmi les mono, di, tri, tétra, penta, hexa, hepta, octo, et nona-hydroxystilbènes, et de préférence parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2, 3',4,4',6-pentahydroxystilbène,
le 2,2',4,4',6,6'-hexahydroxystilbène,
et les mélanges de ces composés,
et plus préférentiellement, la composition contient du 3,4',5-trihydroxystilbène.

7. Composition selon l'une quelconque des revendications précédentes, **caractérisée en ce qu'**elle contient ledit (lesdits) hydroxystilbène(s) en une teneur allant de 0,01 à 10 % en poids, de préférence de 0,05 à 5 % en poids, plus préférentiellement de 0,1 à 2 % en poids, par rapport au poids total de la composition.

8. Composition selon l'une quelconque des revendications précédentes, **caractérisée en ce que** sa teneur en eau va de 30 à 55% en poids, par rapport au poids total de la composition, et de préférence de 30 à 50% en poids.

9. Composition selon l'une quelconque des revendications précédentes, **caractérisée en ce qu'**elle comprend en outre un ou plusieurs solvants organiques choisis parmi les alcools comprenant de 1 à 7 atomes de carbone ; de préférence parmi les monoalcools aliphatiques ou aromatiques en C₁-C₇, les polyols et les éthers de polyols en C₃-C₇; mieux choisis parmi les monoalcools aliphatiques comprenant de 1 à 7 atomes de carbone, plus préférentiellement comprenant de 2 à 4 atomes de carbone.

10. Composition selon la revendication précédente, **caractérisée en ce qu'**elle comprend le (les) solvant(s) organique(s) en une teneur allant de 5 à 70% en poids, de préférence de 10 à 68% en poids, plus préférentiellement de 20 à 65% en poids, et mieux de 30 à 60% en poids, par rapport au poids total de la composition.

11. Composition selon l'une quelconque des revendications précédentes, **caractérisée en ce qu'**elle comprend en outre un ou plusieurs polymère(s) fixant(s), de préférence choisi(s) parmi les polymères fixants non ioniques et les polymères fixants anioniques, mieux parmi les polymères fixants anioniques, en une teneur allant de 0,01 à 10% en poids, de préférence de 0,05 à 5% en poids, et plus préférentiellement de 0,1 à 2% en poids, mieux de 0,2 à 1% en poids, par rapport au poids total de la composition.

12. Procédé de traitement cosmétique non thérapeutique des fibres kératiniques humaines et/ou du cuir chevelu, comprenant l'application sur lesdites fibres et/ou sur le cuir chevelu d'une composition telle que définie dans l'une quelconque des revendications précédentes, pour induire et/ou stimuler la croissance des fibres kératiniques humaines telles que les cheveux et les cils, et/ou freiner leur chute.

13. Utilisation cosmétique non thérapeutique de la composition selon l'une quelconque des revendications 1 à 11, pour induire et/ou stimuler la croissance des fibres kératiniques humaines telles que les cheveux et les cils, et/ou freiner leur chute.
